Europäisches Patentamt

European Patent Office

Office européen des brevets

(11) Publication number: **0 215 726**
**A2**

(12) **EUROPEAN PATENT APPLICATION**

(21) Application number: **86630131.0**

(22) Date of filing: **19.08.86**

(51) Int. Cl.⁴: **A 61 N 1/40, A 61 F 2/00**

(30) Priority: **19.08.85 AU 2025/85**

(43) Date of publication of application: **25.03.87**
**Bulletin 87/13**

(84) Designated Contracting States: **DE FR GB**

(71) Applicant: **THE UNIVERSITY OF MELBOURNE, Grattan Street, Parkville Victoria 3052 (AU)**

(72) Inventor: **Clark, Graeme M., 13 Bannon Road, Eltham Victoria 3095 (AU)**
Inventor: **Franz, Burkhard Karl-Heinz Gunther, 24 Karwitha Street, Vermont Victoria 3133 (AU)**

(74) Representative: **Schmitz, Jean-Marie et al, OFFICE DENNEMEYER S.à.r.l. P.O. Box 1502, L-1015 Luxembourg (LU)**

(54) **Prosthetic electrode array.**

(57) The invention provides an electrode suitable for biological implantation comprising support means and at least one electrode mounted on the support means which support means includes one or more discontinuities in the structure, shape or material of the support means positioned near one end of the support means to facilitate deflection of that end of said support means on encounting an obstacle during the implantation procedure. The invention provides an electrode array for prosthetic implantation, comprising a biologically inert support member having an inner end and an outer end, a plurality of electrodes supported by said support member at predetermined space distances along a portion of the length of said support member.

FIELD OF THE INVENTION

This invention relates to electrode arrays for use with prostheses such as a hearing prosthesis in which the electrode array is implanted in the cochlea of the patient.

BACKGROUND OF THE INVENTION

In Australian Patent 529974 (AU-B46563/79) there is described an electrode array suitable for implantation in the human cochlea to enable stimulation of the auditory nerves by electrical currents applied to the electrodes in the array. The electrode array described in the above patent has been the subject of commercial development by Nucleus Limited of 1 Woodcock Place, Lane Cove, New South Wales and is currently available as part of a hearing prosthesis package being marketed by Nucleus Limited.

Similar electrode arrays are also used in other prosthetic applications, for example, see United States patents 3752939 Bartz, 3995623 Blake et al and 4073283 Bradley et al.

It has been determined that electrodes of the type presently used for implantation in the human cochlea may cause damage to structures within the cochlea, see for example, "Multichannel Intracochlear Elctrodes: Mechanism of Insertion Trauma" by David W. Kennedy M.D. (Proceedings of the Triologic Meeting in Otolaryngology Palm Beach Florida May 1986). This can occur because the tip of the electrode punctures or abrades the structures, or the shaft buckles and causes a tear of soft tissues or fracture of the bony tissues. We have observed in human temporal bones that the electrode tip may cause damage to tissues because the outer wall of the inner ear is slightly splayed outwards. This causes the tip to be directed upwards towards the important soft tissues lying across the middle of the cochlear turn. Similar problems can occur with moulded electrode arrays of the type shown in United States patent 3752939.

BRIEF SUMMARY OF THE INVENTION

It is the object of the present invention to provide an improved electrode suitable for cochlear implantation, but not restricted thereto, in which the above problem is at least substantially ameliorated.

In its broadest form, the invention provides an electrode suitable for biological implantation comprising support means and at least one electrode mounted on the support means, characterized in that said support means includes one or more discontinuities in the structure, shape or material of the support means positioned near one end of the support means to facilitate deflection of that end of said support means on encounting an obstacle during the implantation procedure.

More particularly, the invention provides an electrode array for prosthetic implantation, comprising a biologically inert support member having an inner end and an outer end, a plurality of electrodes supported by said support member at predetermined space distances along a portion of the length of said support member, conducting wires associated with each electrode and passing through the outer end of said support member, and at least one discontinuity in the structure, shape or material of said support member, said discontinuity being positioned such that the inner end of said support member may be deflected on encountering an obstacle during the implantation procedure.

In the case of an electrode suitable for cochlear implantation, the discontinuity is preferably positioned within the front one third of the length of the support member. In the case of an electrode array of the order of 25 mm long, the discontinuity is located in a portion of the inner end of the support member which is about eight to ten mm long. In a particularly preferred form, a first discontinuity is located from 0.5 to 2 mm from the inner end of the support member. The discontinuity may take the form of a narrowing or neck formed in the support material, an element of more flexible material forming part of the support member, for example interposed between the inner end and the body of the support member, or forming the free end of the support member, or a combination of both. More than one discontinuity may be provided.

Studies on the movement of prototype electrode arrays embodying the above invention in human temporal bones have shown that the invention reduces the chance of damage to important structures because the inner end of the support member is directed downwardly away from these structures as the electrode array passes around the cochlear turn. The downward movement would seem due to the fact that the section in front of the discontinuity meets frictional forces on the outer wall before the back section. Alternatively the creation of a discontinuity enables surgical manoeuvres to carried out more effectively, for example, rotation of the electrode, to similarly direct the tip away from important structures and to enable it to pass more easily around the cochlear turn.

A comparison of an electrode embodying the invention with a conventional electrode was conducted using a skeletonised human cochlea which allowed direct observation of the inserted electrodes passing the cochlea turns. While the electrode designed according to the invention allowed the array to pass through the cochlea turns without causing any damage to the structures of the cochlea and while passing through the cochlea turns staying well below the partition above, the conventional electrode penetrated the partition of the cochlea and caused a tear in the spiral ligament and the basilar membrane.

BRIEF DESCRIPTION OF THE DRAWINGS

Several preferred embodiments in the invention will now be described with reference to the accompanying drawings in which:

Figure 1 is a schematic elevation of the inner end of a support member for an electrode array embodying the invention;

Figure 2 is a schematic elevation of the inner end of a support member according to an alternative embodiment of the invention; and

DESCRIPTION OF PREFERRED EMBODIMENTS

Figure 3 is a schematic elevation of the inner end of a still further embodiment of the invention.

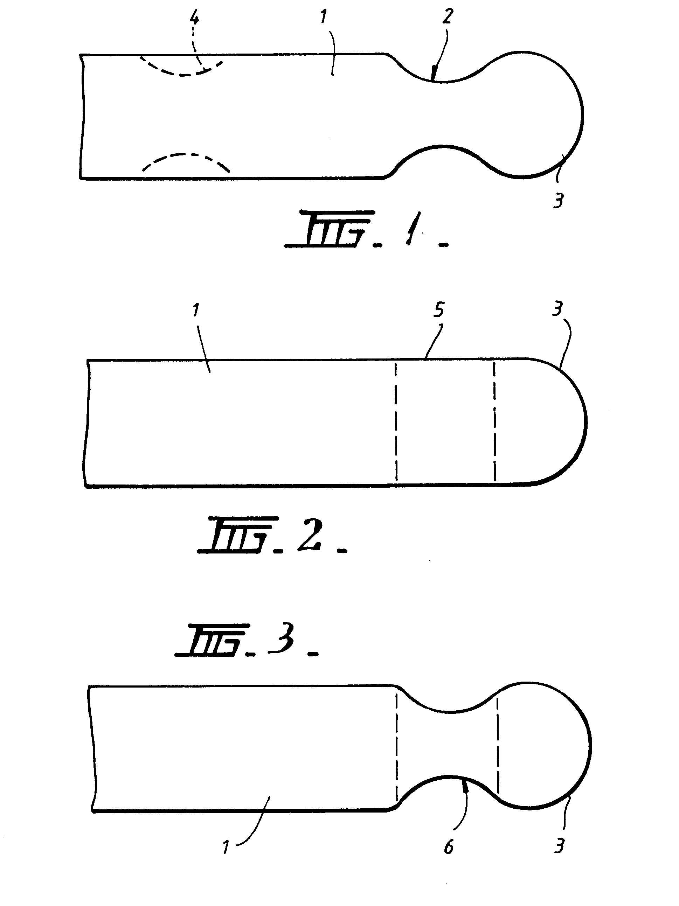

Referring firstly to Figure 1 of the drawings, a support member 1 for an array of electrodes (not shown) is formed with a narrowing or neck 2 from 0.025 to 0.1 mm deep, 0.1 to 0.5 mm wide and located from 0.5 to 2 mm from the inner ends 3 of the support member 1. The support member is preferably made from Silastic 4-4210 and the diameter of the inner end 3 is approximately 0.4 mm. Although not shown in the drawings, the support member 1 is tapered and is approximately 0.6 mm in diameter at its outer end. The support member is approximately 25 mm long and supports twenty electrodes in the form of conductive bands arranged in the manner described in our Australian patent number 529974. However, the invention is equally applicable to electrode arrays having other suitable electrode structures.

If desired, a second discontinuity 4 may be provided and it is preferred that both discontinuities should be located within the inner most one third of the length of the support member 1, that is, approximately 8 to 10 mm from the inner end 3 of the support member 1. The above described arrangement makes it possible for the electrode array to pass around the cochlear turn without damage to the sensitive structures.

Although Silastic 4-4210 has been specified above, Silastic A or medical grade Silastic may be used as the material of the support member 1.

Referring now to Figure 2 of the drawings, a similar result may be achieved by interposing a short element 5 of biologically inert material having a different mechanical property between the body of the support member 1 and the inner end 3. In the embodiment shown, the body of the support member 1 and the inner end 3 are both made from Silastic 4-4210 while the discontinuity 5 is made from Silastic A. The width of the discontinuity 5 is from 0.1 to 0.5 mm, as in the preceding embodiment. A biocompatible glue may be used to secure the discontinuity 5 in position although it will be appreciated that the two types of Silastic bond readily when mixed for extrusion.

In an alternative embodiment not shown in the drawings, the inner end of the support member is made from Silastic A or the like. The end portion extending from the second broken line defining discontinuity 5 may be made entirely from Silastic A to achieve improved results.

In the embodiment of Figure 3, the embodiments of Figures 1 and 2 are effectively combined. Thus, a short section of Silastic A 6 having a necked form as shown in Figure 1 of the drawings is glued between the body of the support member 1 and its inner end 3. Again the dimensions are similar to those of the discontinuity shown in Figure 1 of the drawings.

It will be appreciated that a number of discontinuities of any one or a combination of the above types may be provided along the length of the support member 1. Although the preferred embodiments described above are applicable to so-called free-fitting electrode arrays, the invention is equally applicable to moulded electrode arrays of the type described in United States patent 3752939. Similarly, although the dimensions and materials stipulated above are presently preferred, it will be appreciated that the positioning of a discontinuity at any suitable position near the inner end of the electrode array will improve the

0215726

implantability of the electrode array and serve to ameliorate the problem described above.

CLAIMS:

1. An electrode suitable for electrode suitable for biological implantation comprising support means and at least one electrode mounted on the support means, characterized in that said support means includes one or more discontinuities in the structure, shape or material of the support means positioned near one end of the support means to facilitate deflection of that end of said support means on encounting an obstacle during the implantation procedure.

2. An electrode array for prosthetic implantation, comprising a biologically inert support member having an inner end and an outer end, a plurality of electrodes supported by said support member at predetermined space distances along a portion of the length of said support member, conducting wires associated with each electrode and passing through the outer end of said support member, and at least one discontinuity in the structure, shape or material of said support member, said discontinuity being positioned such that the inner end of said support member may be deflected on encountering an obstacle during the implantation procedure.

3. The electrode array of claim 2, wherein said discontinuity is positioned within the first one third of the length of said support member.

4. The electrode array of claim 3, wherein said support member is about 25mm long, said discontinuity in a portion of the inner end of the support member which is about eight to ten mm long.

5. The array of claim 1, 2 or 3, wherein said discontinuity comprises a neck formed in the surface of said support member.

6. The array of claim 1, 2 or 2, wherein said discontinunity comprises an element of material more flexible than the material of said support member.

7. The electrode array of claim 4, wherein said discontinuity is located from 0.5 to 2mm from the inner end of the support member and comprises a neck in said support member from 0.1 to 0.15mm wide and 0.025 to 0.1mm deep.

8. The electrode array of claim 4, wherein said discontinuity comprises a neck formed in the surface of said member and comprises an element of material more flexible than the material of the support member approximately 0.1 to 0.5mm wide.

9. The electrode array of claim 6, wherien said element is nicked to a depth of 0.002 to 0.1mm.

_FIG. 1._

_FIG. 2._

_FIG. 3._